# FASCICULE DE BREVET EUROPEEN

(11) **EP 2 935 457 B1**
(45) Date de publication et mention de la délivrance du brevet: **19.10.2016**
(21) Numéro de dépôt: 13818265.4
(22) Date de dépôt: 09.12.2013
(51) Int. Cl.: C08L 33/08, A61K 9/16, C11D 3/50, C08F 220/28, C08L 33/10, C08K 5/01, C08L 33/14

(54) **MICROPARTICULES D'AGENT ACTIF**
WIRKSTOFF-MIKROPARTIKEL
ACTIVE INGREDIENT MICROPARTICLES

(30) Priorité: 20.12.2012 FR 1262499; 21.12.2012 US 201261740482 P
(43) Date de publication de la demande: 28.10.2015
(73) Titulaire: COATEX, 69730 Genay (FR)
(72) Inventeur: CHAMPAGNE, Clémentine, F-69300 Caluire-et Cuire (FR); SUAU, Jean-Marc, F-69480 Lucenay (FR); GUERRET, Olivier, F-46170 Pern (FR)
(74) Mandataire: Balmefrezol, Ludovic Francis Pierre
(86) Numéro de dépôt international: PCT/FR2013/052996
(87) Numéro de publication internationale: WO 2014/096622

(56) Documents cités:
- WO-A1-2008/058833
- FR-A1- 2 775 441
- FR-A1- 2 916 655

## Description

### DOMAINE DE L'INVENTION

La présente invention concerne le domaine des encapsulations d'agents actifs, tout particulièrement les encapsulations d'agents actifs au moyen de copolymères acryliques de type HASE.

### ARRIERE PLAN DE L'INVENTION

Les techniques de micro-encapsulation sont de plus en plus développées et amplement utilisées dans les domaines de l'industrie pharmaceutique, cosmétique, textile, agroalimentaire, agrochimique, des détergents, de la peinture. De nombreux agents actifs, hydrophiles ou hydrophobes, peuvent être encapsulés, tels que des molécules odorantes, des principes actifs pharmaceutiques, des agents cosmétiques, des pigments photochromes ou photoluminescents, etc.

L'encapsulation est une méthode efficace pour protéger un agent actif de l'environnement extérieur (pH, lumière, oxygène, humidité...) empêchant ainsi des phénomènes indésirables telles que les réactions induites par la lumière et/ou l'oxygène.

L'encapsulation permet également de protéger un agent actif d'autres molécules et ainsi évite des problèmes d'incompatibilité. L'encapsulation permet également de limiter la dégradation d'un agent actif pendant les procédés de transformation ou de stockage d'un produit. Elle peut également rendre la manipulation ou le stockage de ces agents actifs plus facile.

De nombreuses méthodes d'encapsulation sont connues. Ainsi, il a été proposé d'encapsuler des agents actifs par polymérisation in-situ, par extraction de solvant ou encore par coacervation.

Par exemple, il a été proposé d'encapsuler des agents actifs tels que des principes actifs pharmaceutiques ou des substances agrochimiques au moyen de polymères acryliques de type HASE.

FR2916655 divulgue une méthode pour formuler des principes actifs odorants de manière à les protéger lors d'une application sur la peau ou sur un autre support tel qu'un textile ou un mur et de façon à contrôler la libération progressive desdits principes actifs après leur application; elle repose sur l'utilisation d'émulsions acryliques de type HASE à un pH supérieur à 5.

FR2775441 divulgue une matrice destinée à former des microparticules comportant un principe actif et comprenant un composé hydrophobe solide, la matrice ayant une température de fusion entre 25 et 85°C.

WO 2008/058833 divulgue des particules comportant un composé assouplissant pour textiles encapsulées dans un matériau ayant une température de transition de phase de 26 à 39 °C comme des praffines.

Un des problèmes majeurs lié aux encapsulations demeure le contrôle de la libération ultérieure de l'agent actif encapsulé. Pour certaines applications, il est important que la libération de l'agent actif puisse être déclenchée au moment désiré afin que celui-ci remplisse ses fonctions. La libération de l'agent doit donc pouvoir intervenir en réponse à un stimulus. Pour d'autres applications, il est important de retarder la libération de l'agent actif (libération contrôlée). Par exemple, lorsque l'agent actif est un agent odorant, particulièrement un agent odorant instable chimiquement ou vulnérable aux agressions extérieures, il est important de s'assurer que celui-ci soit stable dans la formulation initiale (lessive, peinture, formulations parfumées) mais également stable après application sur une surface (textile, mur, peau). Une augmentation de la rémanence est donc typiquement recherchée. Les procédés d'encapsulation proposés à ce jour n'offrent généralement pas de flexibilité quant au mode de déclenchement ou de contrôle de la libération de l'agent actif, les capsules formées libérant généralement l'agent actif en réponse à un seul stimulus. Ainsi, en fonction des domaines d'application (par exemple, l'industrie pharmaceutique, cosmétique, textile, agroalimentaire, agrochimique, des détergents, de la peinture), du type d'agents actifs, du mode de libération désiré, il est nécessaire de choisir un procédé d'encapsulation adapté.

Un besoin existe donc pour la mise au point de particules pouvant libérer un agent actif en réponse à différents stimuli ou pouvant retarder la libération de celui-ci. Un besoin existe également pour la mise au point d'un procédé polyvalent de protection d'un agent actif, c'est-à-dire un procédé qui permette de protéger différents types d'agents actifs et permette leur libération déclenchée et/ou leur libération contrôlée lorsque l'une ou l'autre est recherchée. Ce procédé sera de préférence respectueux de l'environnement.

### BREVE DESCRIPTION DE L'INVENTION

La présente invention concerne des microparticules à enveloppe polymérique ainsi que des dispersions aqueuses comprenant ces microparticules. Les microparticules à enveloppe polymérique comprennent: :
a. au moins un copolymère acrylique de type HASE ;
b. au moins un matériau à changement de phase solide-liquide présentant une température de transition de phase variant de 20 à 90°C ;
c. au moins un agent actif.La présente invention concerne également l'utilisation de ces microparticules ou des dispersions aqueuses les comprenant pour libérer un agent actif en réponse à un changement de pH, un changement de température et/ou à des frictions.

La présente invention concerne également l'utilisation de ces microparticules ou des dispersions aqueuses les comprenant pour la préparation de produits cosmétiques, agrochimiques, peintures, textiles, détergents, papier.

La présente invention concerne également un procédé de préparation d'une dispersion aqueuse de microparticules comprenant les étapes suivantes :
a) préparation d'une solution aqueuse comprenant :
   - au moins un copolymère acrylique de type HASE solubilisé dans ladite solution aqueuse au moyen d'une base,
   - au moins un agent actif ; et
   - au moins un matériau à changement de phase solide-liquide, ledit matériau à changement de phase solide-liquide étant présent, ou ayant été introduit, dans la solution aqueuse à une température supérieure à sa température de transition de phase ; et
b) coacervation du polymère acrylique de type HASE pour conduire auxdites dispersions aqueuses de microparticules.

Enfin la présente invention concerne un procédé de préparation de particules solides à partir
d'une dispersion aqueuse de microparticules par séchage.

### BREVE DESCRIPTION DES FIGURES

La figure 1 représente des microparticules qui n'appartiennent pas à la présente invention (A) et des microparticules de la présente invention (B, C et D) observées par microscopie confocale.
La figure 2 représente des microparticules de la présente invention observées par microscopie confocale.
La figure 3 représente la variation de D_{50%} / D⁰_{50%} des microparticules (en ordonnées) en fonction du gradient de cisaillement appliqué (DG/dt en 1/s) (en abscisses) pour différentes teneurs massiques en copolymère acrylique de type HASE.
La figure 4 représente la variation de D_{50%} / D⁰_{50%} des microparticules (en ordonnées) en fonction du gradient de cisaillement appliqué (DG/dt en 1/s) (en abscisses) pour des ratios paraffine/limonène variables.
La figure 5 représente la variation de D_{50%} / D⁰_{50%} des microparticules (en ordonnées) en fonction du gradient de cisaillement appliqué (DG/dt en 1/s) (en abscisses) pour des pH variables.
La figure 6 représente des images de microscopie d'un tissu de coton plongé dans une solution contenant 1.5% de particules de l'essai B puis séché à température ambiante.

### DEFINITION

Dans la description de la présente invention, le terme « HASE » est un acronyme pour « Hydrophobically modified Alkali-soluble Emulsions ».

Dans la description de la présente invention, l'expression « copolymère acrylique de type HASE » désigne des copolymères linéaires ou réticulés, comprenant des groupements acides et des groupements hydrophobes. Les copolymères de type HASE résultent de la copolymérisation de monomères anioniques, tels que les acides (méth)acryliques, de monomères non ioniques hydrophobes et de macromonomères associatifs hydrophobes. A faible pH (typiquement inférieur à 5) et sans l'addition d'une base, le copolymère acrylique de type HASE est insoluble dans l'eau et est présent sous forme de latex. Quand une base est ajoutée, les groupements anioniques sont neutralisés et le copolymère se solubilise dans l'eau.

Dans la description de la présente invention, l'expression « matériau à changement de phase solide-liquide» désigne un matériau qui a la capacité de changer d'état, de manière réversible, dans une plage de température variant de 20 à 90°C. Le matériau à changement de phase entrant dans la composition des microparticules de la présente invention est solide à une température inférieure à sa température de transition de phase et liquide à une température supérieure à sa température de transition de phase. Par « température de transition de phase» est désignée la température de fusion du matériau ou la température correspondant à la transition solide - liquide du matériau à changement de phase.

Dans la description de la présente invention, le terme « agent actif » ou « principe actif » désigne tout composé présentant un intérêt à être encapsulé.

Dans la description de la présente invention, à moins qu'il n'en soit indiqué autrement, les pourcentages exprimés représentent des pourcentages en poids et sont exprimés par rapport au poids total de l'élément de référence. Par exemple, lorsqu'il est indiqué qu'un copolymère comprend 10% d'un monomère, il est entendu que le copolymère comprend 10% en poids de ce monomère par rapport au poids total de ce copolymère.

Dans la description de la présente invention, l'expression « au moins un » désigne un ou plusieurs composés (par exemple : un ou plusieurs copolymères acryliques de type HASE, un ou plusieurs matériaux à changement de phase, un ou plusieurs agents actifs) tels qu'un mélange de 2 à 5 composés.

Dans la description de la présente invention, l'expression « microparticules » désignent des particules ayant une taille moyenne variant de 0.5 µm à quelques dizaines de micromètres, tels que de 0.5 à 100 µm, ou de 1 à 70 µm, ou de 1 à 40 µm. Lorsque les microparticules sont des particules sphériques, la taille moyenne des particules désigne le diamètre moyen des particules. Lorsque les particules ne sont pas sphériques, c'est-à-dire qu'elles présentent une plus longue dimension et une plus courte dimension, la taille moyenne des particules désigne la taille de la plus longue dimension des particules. La taille des particules peut être mesurée selon des méthodes bien connues de l'homme du métier, telles que par granulométrie laser.

Par « microparticules à enveloppe polymérique » ou « microparticules composites » ou « microcapsules », on entend des microparticules présentant une enveloppe externe constituée d'un copolymère selon l'invention et renfermant un agent actif selon l'invention.

Dans la description de la présente invention, les lettres « n », « m » et « p » désignent des nombres entiers.

### DESCRIPTION DETAILLEE DE L'INVENTION

Les microparticules à enveloppe polymérique de la présente invention permettent de protéger de manière efficace un agent actif. De manière avantageuse, les microparticules à enveloppe polymérique de la présente invention permettent une libération de l'agent actif en réponse à un changement de pH, un changement de température et/ou une augmentation des contraintes mécaniques (frictions). Le mode de libération de l'agent actif est choisi en fonction de la nature de l'agent actif et de son utilisation ultérieure. La résistance mécanique des microparticules à enveloppe polymérique de la présente invention pouvant être contrôlée, il devient possible de limiter la dégradation des microparticules dans le temps et ainsi, par exemple, de contrôler la diffusion passive de l'agent actif au cours du temps.

Les microparticules à enveloppe polymérique de la présente invention peuvent se présenter sous forme de dispersion aqueuse ou elles peuvent se présenter sous forme de microparticules solides.

Les microparticules à enveloppe polymérique de la présente invention comprennent :
- au moins un copolymère acrylique de type HASE,
- au moins un matériau à changement de phase solide-liquide présentant une température de transition de phase variant de 20 à 90°C, et
- au moins un agent actif.

### Copolymère acrylique de type HASE

Les copolymères acryliques de type HASE forment l'enveloppe externe des microparticules de la présente invention.

Selon des modes de réalisation de l'invention, les copolymères acryliques de type HASE entrant dans la composition des microparticules de la présente invention comprennent les monomères suivants :
a) au moins un monomère anionique présentant une fonction vinylique polymérisable et un groupement carboxyle;
b) au moins un monomère hydrophobe non ionique présentant une fonction vinylique polymérisable ; et
c) au moins un macromonomère associatif oxyalkylé présentant une fonction vinylique polymérisable et une chaîne hydrocarbonée hydrophobe.

Les monomères anioniques présentant une fonction vinylique polymérisable et un groupement carboxyle sont des monomères présentant une charge négative en solution aqueuse basique. Les monomères anioniques présentant une fonction vinylique polymérisable et un groupement carboxyle sont, par exemple, choisis parmi l'acide acrylique et/ou l'acide méthacrylique.

Les monomères hydrophobes non ioniques présentant une fonction vinylique polymérisable sont des monomères présentant ni charge positive ni charge négative en solution aqueuse. Les monomères hydrophobes non ioniques présentant une fonction vinylique polymérisable sont, par exemple, choisis parmi les esters, les amides ou les nitriles des acides acryliques ou méthacryliques ou parmi l'acrylonitrile, l'acétate de vinyle, le styrène, le méthylstyrène, le diisobutylène, la vinylpyrrolidone ou la vinylcaprolactame. Tout particulièrement, les monomères hydrophobes non ioniques présentant une fonction vinylique polymérisable peuvent être choisis parmi les acrylates d'alkyle en C₁-C₈ ou les méthacrylates d'alkyle en C₁-C₈, tels que l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle ou leurs mélanges. Plus particulièrement, les monomères hydrophobes non ioniques présentant une fonction vinylique polymérisable peuvent être choisis parmi l'acrylate d'éthyle, l'acrylate de butyle, le méthacrylate d'éthyle ou leurs mélanges

Le macromonomère associatif oxyalkylé présentant une fonction vinylique polymérisable et une chaîne hydrocarbonée hydrophobe peut présenter la formule (I) suivante :

(I) R-A-R'

dans laquelle :
- A représente une chaîne polymérique constituée de :
   - m motifs d'oxyde d'alkylène de formule -CH₂CHR₁O- avec R₁ représentant un groupement alkyle comprenant de 1 à 4 carbones, par exemple un groupement méthyle ou éthyle, et m variant de 0 à 150,
   - p motifs d'oxyde d'alkylène de formule -CH₂CHR₂O- avec R₂ représentant un groupement alkyle comprenant de 1 à 4 carbones, par exemple un groupement méthyle ou éthyle, et p allant de 0 à 150,
   - n motifs d'oxyde d'éthylène avec n variant de 0 à 150, ou de 10, ou 15, à 150, ou de 10, ou 15, à 100, ou de 15 à 50, ou de 15 à 30,
   dans laquelle m+n+p >4, ou m+n+p ≥5, et dans laquelle les motifs d'oxyde d'alkylène de formule -CH₂CHR₁O-, les motifs d'oxyde d'alkylène de formule -CH₂CHR₂O- et les motifs d'oxyde d'éthylène sont en bloc, alternés ou statistiques ;
- R représente un radical contenant une fonction insaturée polymérisable, appartenant au groupe des esters acrylique, méthacrylique, maléique, itaconique, ou crotonique ; et
- R' représente une chaîne hydrocarbonée linéaire, branchée ou ramifiée comprenant de 6 à 40 atomes de carbone, ou de 7 à 35 atomes de carbone ou de 8 à 20 atomes de carbone ou de 8 à 16 atomes de carbone ou un groupement cycloalkyle ou aryle substitué, ou non substitué, comprenant de 6 à 100 atomes de carbone ou de 6 à 60 atomes de carbone.
Les groupements R₁ et R₂ peuvent être identiques ou différents.

Dans des modes de réalisation particuliers, le macromonomère associatif oxyalkylé présentant une fonction vinylique polymérisable et une chaîne hydrocarbonée hydrophobe présente la formule (I) suivante :

(I) R-A-R'

dans laquelle R, R' et A sont tels que définis ci-dessus, avec n représentant un nombre de motifs d'oxyde d'éthylène variant de 15 à 150 ou de 15 à 50 ou de 15 à 30.

Dans des modes de réalisation particuliers, le macromonomère associatif oxyalkylé présentant une fonction vinylique polymérisable et une chaîne hydrocarbonée hydrophobe présente la formule (I) suivante :

(I) R-A-R'

dans laquelle R représente un radical contenant une fonction insaturée polymérisable appartenant au groupe des esters acryliques ou méthacryliques et A et R' sont tels que définis dans les modes de réalisation décrits ci-dessus.

Dans des modes de réalisation particuliers, le macromonomère associatif oxyalkylé présentant une fonction vinylique polymérisable et une chaîne hydrocarbonée hydrophobe présente la formule (I) suivante :

(I) R-A-R'

dans laquelle R' représente une chaîne hydrocarbonée linéaire, branchée ou ramifiée comprenant de 8 à 20 atomes de carbone ou de 8 à 16 atomes de carbone, de préférence une chaîne hydrocarbonée linéaire de 8 à 18 atomes de carbone, ou de 8 à 14 atomes de carbone, ou une chaîne ramifiée, ou branchée, de 12 à 20 atomes de carbone, ou de 12 à 16 atomes de carbone, et A et R sont tels que définis dans les modes de réalisation décrits ci-dessus.

Dans des modes de réalisation particuliers, le macromonomère associatif oxyalkylé présentant une fonction vinylique polymérisable et une chaîne hydrocarbonée hydrophobe présente la formule (I) suivante :

(I) R-A-R'

dans laquelle A, R et R' sont tels que définis dans les modes de réalisation décrits ci-dessus, avec m et p représentant respectivement 0.

Dans des modes de réalisation particuliers, le macromonomère associatif oxyalkylé présentant une fonction vinylique polymérisable et une chaîne hydrocarbonée hydrophobe présente la formule (I) suivante :

(I) R-A-R'

dans laquelle :
- A est tel que défini dans les modes de réalisation décrits ci-dessus, avec m et p représentant respectivement 0 et n variant de 15 à 150 ou de 15 à 50 ou de 15 à 30 (ainsi A représente une chaîne polymérique constituée de 15 à 150 ou de 15 à 50 ou de 15 à 30 motifs d'oxyde d'éthylène) ;
- R représente un radical contenant une fonction insaturée polymérisable appartenant au groupe des esters acrylique, méthacrylique, maléique, itaconique, ou crotonique, de préférence appartenant au groupe des esters acryliques ou méthacryliques ; et
- R' représente une chaîne hydrocarbonée linéaire, branchée ou ramifiée comprenant de 6 à 40 atomes de carbone, ou de 7 à 35 atomes de carbone ou de 8 à 20 atomes de carbone ou de 8 à 16 atomes de carbone.

Dans des modes de réalisation particuliers, le macromonomère associatif oxyalkylé présentant une fonction vinylique polymérisable et présentant une chaîne hydrocarbonée hydrophobe présente la formule (I) suivante :

(I) R-A-R'

dans laquelle :
- A est tel que défini dans les modes de réalisation décrits ci-dessus, avec m et p représentant respectivement 0 et n variant de 15 à 150 ou de 15 à 50 ou de 15 à 30 (ainsi A représente une chaîne polymérique constituée de 15 à 150 ou de 15 à 50 ou de 15 à 30 motifs d'oxyde d'éthylène) ;
- R représente un radical contenant une fonction insaturée polymérisable appartenant au groupe des esters acryliques ou méthacryliques ; et
- R' représente une chaîne hydrocarbonée linéaire, branchée ou ramifiée comprenant de 8 à 20 atomes de carbone, par exemple une chaîne hydrocarbonée linéaire comprenant de 8 à 18 atomes de carbone ou de 8 à 14 atomes de carbone ou une chaîne alkyle ramifiée, ou branchée, comprenant de 12 à 20 atomes de carbone ou de 12 à 16 atomes de carbone.

Dans des modes de réalisation particuliers, les copolymères acryliques entrant dans la composition des particules de la présente invention comprennent :
a) au moins un monomère anionique présentant une fonction vinylique polymérisable et un groupement carboxyle choisi parmi l'acide acrylique, l'acide méthacrylique ou leur mélange ;
b) au moins un monomère hydrophobe non ionique présentant une fonction vinylique polymérisable choisi parmi l'acrylate d'éthyle, le méthacrylate de méthyle, l'acrylate de butyle ou leurs mélanges ; et
c) au moins un macromonomère associatif oxyalkylé présentant une fonction vinylique polymérisable et présentant une chaîne hydrocarbonée de formule (I) suivante :

   (I) R-A-R'
dans laquelle A, R et R' sont tels que définis dans les modes de réalisation décrits ci-dessus.

Dans des modes de réalisation particuliers, les copolymères acryliques entrant dans la composition des particules de la présente invention comprennent :
a) au moins un monomère anionique présentant une fonction vinylique polymérisable et un groupement carboxyle choisi parmi l'acide acrylique, l'acide méthacrylique ou leur mélange ;
b) au moins un monomère hydrophobe non ionique présentant une fonction vinylique polymérisable choisi parmi l'acrylate d'éthyle, le méthacrylate de méthyle, l'acrylate de butyle ou leurs mélanges ;
c) au moins un macromonomère associatif oxyalkylé présentant une fonction vinylique polymérisable et présentant une chaîne hydrocarbonée hydrophobe de formule (I) :

   (I) R-A-R'

   dans laquelle :
   - A est tel que défini dans les modes de réalisation décrits ci-dessus, avec m et p représentant respectivement 0 et n variant de 15 à 150 ou de 15 à 50 ou de 15 à 30 (ainsi A représente une chaîne polymérique constituée de 15 à 150 ou de 15 à 50 ou de 15 à 30 motifs d'oxyde d'éthylène) ;
   - R représente un radical contenant une fonction insaturée polymérisable, appartenant au groupe des esters acrylique, méthacrylique, maléique, itaconique, ou crotonique, de préférence au groupe des esters acryliques ou méthacryliques ;
   - R' représente une chaîne hydrocarbonée linéaire, branchée ou ramifiée comprenant de 8 à 20 atomes de carbone ou de 8 à 16 atomes de carbone, plus particulièrement une chaîne hydrocarbonée linéaire comprenant de 8 à 18 atomes de carbone, ou de 8 à 14 atomes de carbone, ou une chaîne hydrocarbonée ramifiée ou branchée comprenant de 12 à 20 atomes de carbone ou de 12 à 16 atomes de carbone.

Les copolymères acryliques entrant dans la composition des particules de la présente invention comprennent typiquement:
a) de 20% à 65% ou de 30% à 45% en poids d'au moins un monomère anionique présentant une fonction vinylique polymérisable et un groupement carboxyle ;
b) de 35% à 75% ou de 45% à 60% en poids d'au moins un monomère hydrophobe non ionique présentant une fonction vinylique polymérisable ; et
c) de 0.5% à 15% ou de 1% à 12% en poids d'au moins un macromonomère associatif oxyalkylé présentant une fonction vinylique polymérisable et une chaîne hydrocarbonée hydrophobe, plus particulièrement d'un macromonomère associatif oxyalkylé de formule (I) suivante :

   (I) R-A-R'
dans laquelle A, R et R' sont tels que définis dans les modes de réalisation décrits ci-dessus.

Dans des modes de réalisation particuliers, les copolymères acryliques entrant dans la composition des particules de la présente invention comprennent :
a) de 20% à 65% ou de 30% à 45% en poids d'au moins un monomère anionique présentant une fonction vinylique polymérisable et un groupement carboxyle choisi parmi l'acide acrylique, l'acide méthacrylique ou leur mélange ;
b) de 35% à 75% ou de 45% à 60% en poids d'au moins un monomère hydrophobe non ionique présentant une fonction vinylique polymérisable choisi parmi l'acrylate d'éthyle, le méthacrylate de méthyle, l'acrylate de butyle ou leurs mélanges ; et
c) de 0.5% à 15% ou de 1% à 12% en poids d'au moins un macromonomère associatif oxyalkylé présentant une fonction vinylique polymérisable et présentant une chaîne hydrocarbonée hydrophobe de formule (I) :

   (I) R-A-R'

   dans laquelle :
   - A est tel que défini dans les modes de réalisation décrits ci-dessus, avec m et p représentant respectivement 0 et n variant de 15 à 150 ou de 15 à 50 ou de 15 à 30 (ainsi A représente une chaîne polymérique constituée de 15 à 150 ou de 15 à 50 ou de 15 à 30 motifs d'oxyde d'éthylène) ;
   - R représente un radical contenant une fonction insaturée polymérisable, appartenant au groupe des esters acrylique, méthacrylique, maléique, itaconique, ou crotonique, de préférence au groupe des esters acryliques ou méthacryliques ; et
   - R' représente une chaîne hydrocarbonée linéaire, branchée ou ramifiée comprenant de 8 à 20 atomes de carbone, ou de 8 à 16 atomes de carbone, plus particulièrement une chaîne hydrocarbonée linéaire comprenant de 8 à 18 atomes de carbone, ou de 8 à 14 atomes de carbone, ou une chaîne hydrocarbonée ramifiée, ou branchée, comprenant de 12 à 20 atomes de carbone ou de 12 à 16 atomes de carbone.

Les copolymères acryliques de type HASE entrant dans la composition des particules de la présente invention résultent de la copolymérisation des monomères décrits ci-dessus. Ils peuvent être préparés selon les méthodes décrites dans WO 2011/104599, WO 2011/104600 et EP1778797.

Durant la polymérisation, un agent de transfert de chaînes peut être ajouté pour contrôler le poids moléculaire du copolymère. L'agent de transfert de chaîne peut être choisi parmi les mercaptans tels que l'éthyle mercaptan, n-propyl mercaptan, n-butyl mercaptan, isobutyl mercaptan, t-butyl mercaptan, n-amyl mercaptan, isoamyl mercaptan, t-amyl mercaptan, n-hexyl mercaptan, cyclohexyl mercaptan, n-octyl mercaptan, n-decyl mercaptan ou n-dodecyl mercaptan.

### Matériau à changement de phases

Les particules de la présente invention comprennent au moins un matériau à changement de phase solide-liquide. Le matériau à changement de phase a la capacité de changer d'état, de manière réversible, dans une plage de température variant de 20 à 90°C, ou de 25 à 80°C ou de 35 à 70°C.

Les matériaux à changement de phase entrant dans la composition des particules de la présente invention peuvent être choisis parmi les cires d'origine naturelle ou synthétique. Les cires d'origine naturelle incluent les cires animales, les cires végétales et les cires minérales.

Les cires animales et végétales sont généralement constituées d'un mélange de lipides à longue chaîne hydrocarbonée, tels que les acides gras, les alcools gras ou les esters d'acide gras ou d'alcool gras, voire des éthers. Par « longues chaînes hydrocarbonées », on entend des chaînes hydrocarbonées présentant par exemple de 10 à 40 atomes de carbones. Les cires animales et végétales présentent typiquement une température de transition de phase variant de 25°C à 90°C. Des exemples de cires végétales incluent les cires de carnauba, de candelilla, de sucre de canne, d'Esparto ou le beurre de karité. Des exemples de cires animales incluent les cires d'abeille ou la lanoline.

Les cires minérales, incluant les cires paraffiniques (communément appelées Paraffine), sont généralement constituées d'hydrocarbures saturés à chaîne linéaire comprenant par exemple de 20 à 40 atomes de carbone. Les cires minérales présentent typiquement une température de transition de phase allant de 25°C à 90°C. Des exemples de cire minérale incluent la cérésine, l'ozokerite, les cires de paraffine et les cires microcristallines. Des exemples de cires de paraffine incluent l'heneicosane dont la température de fusion est de 40,5°C, l'eicosane dont la température de fusion est de 36,1 °C et la nonadecane dont la température de fusion est de 32,1°C.

Les cires d'origine synthétique sont généralement constituées de longues chaînes hydrocarbonées dépourvues de groupements fonctionnels. Des exemples de cires d'origine synthétique incluent les polymères à base de polyéthylène et les polymères à base de polyalkylène glycol, tels que les polymères à base de polyéthylène glycol et les polymères à base de polypropylène glycol.

Les matériaux à changement de phase entrant dans la composition des microparticules de la présente invention peuvent être choisis parmi les alcools à longue chaîne hydrocarbonée, par exemple les alcools présentant de 14 à 30 atomes de carbone, ou de 14 à 22 atomes de carbone, tels que l'alcool myristylique, l'alcool cétylique, l'alcool stéarique, l'alcool arachidique, l'alcool béhénique, les acides gras à longue chaîne hydrocarbonée, par exemple les acides présentant de 12 à 30 atomes de carbone ou de 12 à 22 atomes de carbone, tels que l'acide décanoïque, l'acide laurique, l'acide myristique, l'acide palmitique, l'acide stéarique, l'acide arachidique, l'acide béhénique, les esters d'acide gras, tels que les esters d'acide décanoïque, d'acide laurique, d'acide myristique, d'acide palmitique, d'acide stéarique, d'acide arachidique, d'acide béhénique, les éthers gras ou leurs mélanges.

Les matériaux à changement de phase entrant dans la composition des particules de la présente invention seront choisis au regard de l'utilisation ultérieure des particules.

### Agent actif

Les microparticules de la présente invention comprennent au moins un agent actif.

L'agent actif peut être choisi parmi les parfums ou molécules odorantes (par exemple le menthol, le chèvrefeuille, l'huile de cananga, le citronellal, l'aurantiol ou le limonène), les huiles essentielles, les arômes, les opacifiant, les agents hydratants, les agents adoucissants, les agents rafraîchissants, les colorants, les plastifiants, les agents amincissants, les principes actifs pharmaceutiques, les encres, les pigments tels que les pigments photochromes ou photoluminescents, les principes actifs agrochimiques, les antiseptiques, les détergents, les enzymes, les anti-mousses, les agents de blanchiment, les azurants optiques ou les agents antibactériens.

Dans certains modes de réalisation de la présente invention, l'agent actif est un parfum ou une molécule odorante.

### Les microparticules de la présente invention

Dans certains modes de réalisation, les microparticules de la présente invention comprennent, par rapport au poids total des microparticules :
- de 1 à 20%, ou de 1 à 15% ou de 1 à 10% en poids, d'au moins un copolymère acrylique de type HASE tel décrit ci-dessus ;
- de 4 à 95%, ou de 4 à 75% ou de 4 à 50%, en poids, d'au moins un matériau à changement de phase tel que décrit ci-dessus ; et
- de 4 à 95%, ou de 24 à 95% ou de 49 à 95%, en poids d'au moins un agent actif tel que décrit ci-dessus.

Les microparticules de la présente invention peuvent se présenter sous forme de dispersion aqueuse de particules ou elles peuvent se présenter sous forme solide, c'est-dire sous forme de granules (ou microcapsules) solides. Ainsi, la présente invention concerne des dispersions aqueuses comprenant des microparticules telles que décrites ci-dessus et des microparticules solides de composition telle que décrite ci-dessus.

Les dispersions aqueuses de microparticules peuvent comprendre de 1 à 70% en poids de microparticules.

Les microparticules de la présente invention permettent de libérer l'agent actif encapsulé en réponse à un changement de pH, un changement de température et/ou une friction. Il est possible dans le cadre de la présente invention d'ajuster la résistance mécanique des microparticules, de manière à contrôler la libération de l'agent actif.
Par « contrôler la libération de l'agent actif », on entend tout à la fois déclencher la libération de l'agent actif en réponse à un stimulus ou retarder cette libération dans le temps. Ainsi, il est possible de déclencher la libération de l'agent actif au moment désiré en fonction de l'application souhaité ou de l'effet recherché. Egalement, il est possible de rendre les microparticules de la présente invention sensibles à un ou plusieurs stimulus et permettent ainsi de libérer l'agent actif encapsulé au moment désiré.

De manière avantageuse, les microparticules de la présente invention présente une bonne résistance mécanique, voire une résistance mécanique supérieure à celle des particules ne comprenant pas de matériaux à changement de phase. De telles caractéristiques sont particulièrement avantageuses lorsque l'agent actif encapsulé est un parfum ou une molécule odorante. En effet, lorsque l'agent actif est un parfum ou une molécule odorante, on cherche typiquement à augmenter sa rémanence. Les microparticules de la présente invention permettent ainsi une libération contrôlée de l'agent actif encapsulé, leur dégradation étant ainsi retardée.

Les microparticules de la présente invention présentent ainsi une résistance mécanique minimale, telle qu'elles maintiennent leur intégrité après avoir été cisaillées pendant 2 min à 200 s⁻¹ à 25°C selon la méthode décrite dans la section « exemples ». Ainsi, selon cet aspect, les microparticules de la présente invention sont dites « à résistance mécanique contrôlée ».

Les concentrations en copolymère acrylique de type HASE, en matériau à changement de phase et/ou en agent actif peuvent être ajustées afin d'obtenir des microparticules plus ou moins résistantes aux pressions mécaniques ; autrement dit à résistance mécanique supérieure ou égale à la résistance mécanique minimale telle que décrite ci-dessus. En effet, pour certaines applications, il est souhaitable d'obtenir des microparticules libérant l'agent actif sous faibles contraintes mécaniques (faibles pressions mécaniques) alors que pour d'autres applications, il est souhaitable d'obtenir des particules résistant à des contraintes mécaniques plus ou moins fortes.

Les microparticules de la présente invention peuvent être utilisées pour libérer un agent actif en réponse à un changement de pH, à un changement de température et/ou à des frictions.

Les dispersions ou microparticules de la présente invention peuvent être utilisées dans le domaine de la cosmétologie, par exemple pour formuler des lotions, shampoings, crèmes, déodorants, compositions de maquillage, compositions de soin. On cite à cet égard en particulier l'encapsulation des parfums, huiles essentielles, opacifiant, agents hydratants, agents adoucissants, agents rafraîchissants, agents amincissants.

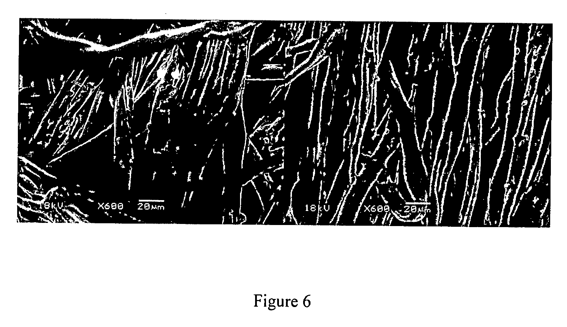

Les dispersions ou microparticules de la présente invention peuvent être utilisées dans le domaine de l'industrie textile, par exemple dans la fabrication de vêtements, collants, bas, gants. On cite à cet égard en particulier l'encapsulation des parfums, des agents amincissants, des agents anti-transpirants, des agents antibactériens. Les dispersions ou microparticules sont alors appliqués sur divers textiles (fig. 6).

Les dispersions ou microparticules de la présente invention peuvent être utilisées dans le domaine des peintures où des colorants, des pigments ou des résines peuvent notamment être encapsulés.
Les microparticules de la présente invention peuvent être utilisées dans le domaine de l'industrie du papier (encapsulation d'encres, de molécules odorantes) ou de la fabrication des détergents (encapsulation d'agents odorants, anti-mousses, blanchissants).

Ainsi les microparticules solides ou dispersions aqueuses de microparticules de la présente invention peuvent être utilisées pour la préparation de produits cosmétiques, agrochimiques, détergents, peintures, textiles, papier.

### Préparation des dispersions aqueuses de particules et particules de la présente invention

Un objet de la présente invention concerne également un procédé de préparation d'une dispersion aqueuse de microparticules, ainsi qu'un procédé de préparation de microparticules à enveloppe polymérique telles que décrites ci-dessus. Les microparticules solides de la présente invention sont généralement produites à partir d'une dispersion aqueuse de microparticules.

Les dispersions aqueuses de microparticules peuvent être préparées par un procédé comprenant les étapes suivantes :
a) préparation d'une solution aqueuse comprenant :
   - au moins un copolymère acrylique de type HASE solubilisé dans ladite solution aqueuse au moyen d'une base, plus particulièrement un copolymère acrylique de type HASE tel que décrit ci-dessus ;
   - au moins un agent actif, plus particulièrement au moins un agent actif tel que décrit ci-dessus ; et
   - au moins un matériau à changement de phase solide-liquide, plus particulièrement au moins un matériau à changement de phase solide-liquide tel que décrit ci-dessus, ledit matériau à changement de phase solide-liquide étant présent, ou ayant été introduit, dans la solution aqueuse à une température supérieure à sa température de transition de phase ; et
b) coacervation du polymère acrylique de type HASE pour conduire auxdites dispersions aqueuses de microparticules.

Lorsque le matériau à changement de phase solide-liquide est dit « présent dans la solution aqueuse à une température supérieure à sa température de transition de phase », il est entendu que la solution aqueuse comprenant le copolymère acrylique de type HASE, la base, l'agent actif et le matériau à changement de phase est à une température supérieure à la température de transition de phase du matériau à changement de phase.

Lorsque le matériau à changement de phase solide-liquide est dit « ayant été introduit dans la solution aqueuse à une température supérieure à sa température de transition de phase », il est entendu que le matériau a été ajouté à une température supérieure à sa température de transition de phase mais que l'eau, la base, le copolymère de type HASE, l'agent actif peuvent être à une température inférieure à cette température de transition de phase. La solution aqueuse finale comprenant le copolymère acrylique de type HASE, la base, l'agent actif et le matériau à changement de phase a ainsi généralement une température inférieure à la température de transition de phase du matériau à changement de phase.

En général, le matériau à changement de phase solide-liquide, seul ou en mélange avec l'agent actif, est chauffé à une température supérieure à sa température de transition de phase et introduit à une température supérieure à sa température de transition de phase dans une solution aqueuse comprenant le copolymère acrylique de type HASE, la base et éventuellement l'agent actif, cette solution aqueuse ayant généralement une température inférieure à la température de transition de phase du matériau à changement de phase.

Dans l'étape a), la base est ajoutée en une quantité permettant de solubiliser le copolymère acrylique de type HASE dans la solution aqueuse.
La base employée dans le procédé est typiquement une base organique ou minérale. La base peut par exemple être choisie parmi la soude, l'ammoniac, la chaux, la potasse et le 2-amino-2-méthyl-1-propanol.
Les articles de Jenkins et al., 2002 (J. Phys. Chem. B 2002, 106, 1195-1204 1195) et Horiuchi et al., 1998 (Can. J. Chem. 76: 1779-1787) décrivent le phénomène de solubilisation des polymères HASE lors de l'ajout d'une base.

Selon des modes de réalisation de l'invention, la quantité de base utilisée pour solubiliser le copolymère est telle que le pH de la solution aqueuse est supérieur ou égal à 6,5, ou supérieur ou égal à 7, ou supérieur ou égal à 7,5.

Selon d'autres modes de réalisation de l'invention, en fonction du type de base utilisée, notamment si la base utilisée est la soude, la quantité de base utilisée pour solubiliser le copolymère est telle que le rapport molaire (nOH⁻/nCOOH) entre le nombre de groupements hydroxyles apportées par la base (nOH⁻) et le nombre de groupements carboxyles portés par le copolymère acrylique de type HASE (nCOOH) soit supérieur à 0.3, ou supérieur à 0.4, ou supérieur à 0.45 et de préférence inférieur à 1.2. Le nombre de groupements carboxyles portés par le copolymère acrylique de type HASE peut être déterminé par des méthodes connues de l'homme du métier, telles que par titration.

Selon des modes de réalisation de l'invention, on prépare la solution aqueuse de l'étape a) sous agitation.

L'étape de coacervation du polymère acrylique de type HASE conduisant aux dispersions aqueuses de microparticules (étape b)) est réalisée une fois que la solution aqueuse est à une température inférieure à la température de transition de phase du matériau à changement de phase, c'est-à-dire après refroidissement de la solution aqueuse.

La coacervation peut être réalisée par addition de sels, tels que des chlorures de sodium, ou alternativement par addition d'un acide.

Dans certaines modes de réalisation de l'étape b) du procédé, la coacervation est réalisée par ajout d'un acide. L'acide employé dans le procédé peut notamment être choisi parmi un acide organique ou minéral. Plus particulièrement, l'acide peut être choisi parmi l'acide phosphorique, l'acide chlorhydrique, l'acide acétique, l'acide citrique, l'acide D-gluconique, l'acide glutamique et l'acide ascorbique.

Dans certaines modes de réalisation de l'étape b) du procédé, la quantité d'acide utilisée pour réaliser la coacervation est telle que le pH de la dispersion est inférieur ou égal à 6,5 ou inférieur ou égal à 6,3.

Dans d'autres modes de réalisation de l'étape b) du procédé, en fonction du type d'acide utilisé, notamment si l'acide utilisé est l'acide acétique, la quantité d'acide ajoutée est telle que le rapport molaire (nH₃O⁺/nCOOH) entre le nombre de protons apportés par l'acide (nH₃O⁺) et le nombre de groupements carboxyles portés par le copolymère acrylique de type HASE (nCOOH) est supérieur à 0.1, ou supérieur à 0.15, ou supérieur à 0.2 et inférieur à 1.

Selon des modes de réalisation de l'invention, l'ajout de sel ou d'acide est réalisé sous agitation.

La coacervation (ou précipitation) du polymère acrylique de type HASE permet de former une enveloppe polymérique qui constitue l'enveloppe externe des microparticules.

Dans certains modes de réalisation, la solution aqueuse comprenant le copolymère acrylique de type HASE solubilisé, l'agent actif et le matériau à changement de phase solide-liquide (étape a)) est préparée selon les étapes suivantes :
a1) préparation d'une solution aqueuse comprenant le copolymère acrylique de type HASE solubilisé au moyen d'une base ;
a2) préparation d'un mélange comprenant l'agent actif et le matériau à changement de phase, ledit mélange étant préparé à une température supérieure à la température de transition de phase du matériau à changement de phase ou étant ensuite chauffé à une température supérieure à la température de transition de phase du matériau à changement de phase ; et
a3) introduction du mélange obtenu à l'étape a2) dans la solution aqueuse obtenue à l'étape a1).

On note que selon ces modes de réalisation, lors de l'étape a3) :
- le mélange obtenu à l'étape a2) se trouve à une température supérieure à la température de transition de phase du matériau à changement de phase, par exemple à une température comprise entre la température de transition de phase du matériau à changement de phase et la température de chauffe du mélange a2) ;
- la solution aqueuse obtenue à l'étape a1) se trouve à une température inférieure à la température de transition de phase du matériau à changement de phase.

Selon des modes de réalisation de l'invention, l'introduction du mélange obtenu à l'étape a2) dans la solution aqueuse obtenue à l'étape a) est réalisée sous agitation.

Dans d'autres modes de réalisation, la solution aqueuse comprenant le copolymère acrylique de type HASE solubilisé, l'agent actif et le matériau à changement de phase solide-liquide (étape a) peut être préparée selon les étapes suivantes :
a1) préparation d'une solution aqueuse comprenant le copolymère acrylique de type HASE solubilisé au moyen d'une base, au moins un agent actif et au moins un matériau à changement de phase ; et
a2) chauffage de la solution aqueuse obtenue à l'étape a1) à une température supérieure à la température de transition du matériau à changement de phase.

Selon des modes de réalisation de l'invention, la solution aqueuse est préparée (étape a1)) sous agitation ou elle est agitée après avoir été préparée. Dans ce cas, elle peut être agitée avant, pendant ou après chauffage (étape a2)), mais avant de réaliser l'étape b).

Selon ces modes de réalisation, l'ordre d'introduction du copolymère acrylique de type HASE, de la base, de l'agent actif et du matériau à changement de phase pour conduire à la solution aqueuse de l'étape a1) est indifférent.

Ainsi, le copolymère acrylique de type HASE, l'eau et la base peuvent être mélangés ensemble dans un premier temps. A ce mélange, peut être ajouté un second mélange comprenant le matériau à changement de phase et l'agent actif, de manière à obtenir la solution aqueuse de l'étape a1).

De manière alternative, le copolymère acrylique de type HASE, l'eau, la base, le matériau à changement de phase et l'agent actif peuvent être mélangés ensemble, sans étape préliminaire de sous-mélange, pour donner la solution aqueuse de l'étape a1).

Ainsi, selon la présente invention, les dispersions aqueuses de particules peuvent être préparées par un procédé comprenant les étapes suivantes :
a1) préparation d'une solution aqueuse comprenant une base et au moins un copolymère acrylique de type HASE, plus particulièrement un copolymère acrylique de type HASE tel que décrit ci-dessus ;
a2) préparation d'un mélange comprenant au moins un agent actif et au moins un matériau à changement de phase, ledit mélange étant préparé à une température supérieure à la température de transition de phase du matériau à changement de phase ;
a3) introduction du mélange obtenu à l'étape a2) dans la solution aqueuse obtenue à l'étape a1); et
b) coacervation du copolymère acrylique de type HASE pour conduire aux dispersions aqueuses de particules.

De manière alternative, les dispersions aqueuses de particules de la présente invention peuvent être préparées par un procédé comprenant les étapes suivantes :
a1) préparation d'une solution aqueuse comprenant une base, au moins un agent actif, au moins un matériau à changement de phase et au moins un copolymère acrylique de type HASE, plus particulièrement un copolymère acrylique de type HASE tel que décrit ci-dessus ;
a2) chauffage de la solution aqueuse obtenue à l'étape a1) à une température supérieure à la température de transition du matériau à changement de phase ; et
b) coacervation du copolymère acrylique de type HASE pour conduire aux dispersions aqueuses de particules.

Des microparticules solides peuvent être obtenues après séchage des dispersions de microparticules obtenues à l'étape b).

Le copolymère acrylique de type HASE, le matériau à changement de phase et l'agent actif employés dans les procédés de la présente invention peuvent être tels que décrits dans la description de la présente invention.

Les procédés mis en oeuvre pour la préparation des microparticules de la présente invention sont respectueux de l'environnement puisqu'il n'est pas fait usage de solvants organiques.

### Procédé de formulation d'un agent actif

Dans un aspect, la présente invention concerne un procédé de formulation d'un agent actif comprenant la préparation de microparticules comprenant au moins un copolymère acrylique de type HASE, au moins un matériau à changement de phase et au moins un agent actif, ladite formulation permettant une libération contrôlée de l'agent actif.

### EXEMPLES

### Réparation du copolymère acrylique HASE 1

Le copolymère acrylique selon l'invention est préparé selon des méthodes connues de l'homme du métier au moyen d'un agent de transfert de chaînes du type mercaptan.

Ce copolymère est constitué de :
35,5 % en poids d'acide méthacrylique,
52,4 % en poids d'acrylate d'éthyle,
12,0 % en poids d'un macromonomère de formule (I) dans laquelle :
   - m et p = 0,
   - n = 30,
   - R représente un ester méthacrylique,
   - R' représente une chaîne hydrocarbonée branchée comprenant de 12 atomes de carbone.

### Réparation d'une dispersion de microparticules selon la présente invention (ref.1)

### Préparation d'une solution aqueuse

7,5 g du copolymère HASE 1 (polymérisé à 30,8% dans l'eau), sont solubilisés dans 61.9 g d'eau en présence de 2.9 g de soude à 10% par agitation dans un mixeur à une température de 40°C. Dans ce cas, on note que nOH⁻ = 7,25.10⁻³ et nCOOH = 9,5.10⁻³. Le pH de la solution se situe aux alentours de pH 8,5.

15,75 g de paraffine commercialisée par la société Sigma-Aldrich sous la référence Paraffin wax 327204 (T_{f} = 53-57°C) sont mélangés avec 47,25 g de limonène. Le mélange est agité et chauffé à une température d'environ 80°C.

A l'aide d'une pompe péristaltique, le mélange liquide paraffine-limonène (à une température comprise entre Tf et 80°C) est introduit dans la solution aqueuse de copolymère HASE 1.

### Coacervation

6,44 g d'une solution de H₃PO₄ à 4% sont ajoutés à la solution aqueuse de copolymère HASE 1 à laquelle a été ajouté le mélange paraffine-limonène. Le pH est mesuré.

Les particules obtenues sont caractérisées par granulométrie laser au moyen du Mastersizer 2000 de Malvern (D_{50%} : diamètre correspondant à 50% de la fréquence cumulée en volume de particules). Les résultats sont présentés dans le tableau 1.

### Préparation d'une dispersion de microparticules selon la présente invention (ref.2)

### Préparation d'une solution aqueuse

15,75g de paraffine commercialisée par la société Sigma-Aldrich sous la référence Paraffin wax 327204 (T_{f} = 53-57°C) sont mélangés avec 47,25g de limonène et l'ensemble est chauffé à 80°C.

En parallèle, 7.5 g du copolymère HASE 1 (polymérisé à 30,8% dans l'eau), sont solubilisés dans 61.9 g d'eau en présence de 2.9 g de soude à 10% par agitation dans un mixeur à une température de 40°C. Dans ce cas, on note que nOH⁻ = 7,25.10⁻³ et nCOOH = 9,5.10⁻³. Le pH de la solution se situe aux alentours de pH 8,5.

La solution aqueuse du copolymère HASE 1 et le mélange paraffine-limonène sont mélangés et chauffés à 80°C.

L'ensemble est mélangé à l'aide d'un mixeur et on laisse la température diminuer progressivement.

### Coacervation

Lorsque le mélange atteint 40°C, 6,44g d'une solution de H₃PO₄ à 4% est ajoutée. Le Ph est mesuré.

Les particules obtenues sont caractérisées par granulométrie laser au moyen du Mastersizer 2000 de Malvern (D_{50%} : diamètre correspondant à 50% de la fréquence cumulée en volume de particules). Les résultats sont présentés dans le tableau 1.

**Tableau 1 : Granulométrie des particules. Etude de la variation de la teneur en cire, en agent actif, en copolymère HASE1 et étude de la variation de la nature de la cire, de la nature de l'agent actif**

| **Ref.** | **Teneur massique en HASE1 (%)** | **Teneur massique en cire (%)** | **Teneur massique en PA (%)** | **pH final** | **D_{50%} (µm)** |
|---|---|---|---|---|---|
| 1 | 3,5 | 24,1 | 72,3 | 6,2 | 2,76 |
| 2 | 3,5 | 24,1 | 72,3 | 6,7 | 3,66 |

Les dispersions de particules présentées ci-dessous (tableau 2) ont été préparées selon le procédé mis en oeuvre pour préparer la réf. 1.

Les particules obtenues sont caractérisées par granulométrie laser au moyen du Mastersizer 2000 de Malvern (D_{50%} : diamètre correspondant à 50% de la fréquence cumulée en volume de particules). Les résultats sont présentés dans le tableau 2.

**Tableau 2 : Granulométrie des particules.**

| **Essai** | **Cire** | **Agent actif** | **Teneur massique en HASE1 (%)** | **Teneur massique en cire (%)** | **Teneur massique en Agent actif (%)** | **pH final** | **D_{50%} (µm)** |
|---|---|---|---|---|---|---|---|
| A | - | Limonène | 3,5 | 0 | 96,5 | 6,3 | 1,94 |
| B | Paraffine 1 | Limonène | 3,5 | 24,1 | 72,3 | 6,3 | 2,80 |
| C | Paraffine 1 | Limonène | 3,5 | 24,1 | 72,3 | 6,2 | 2,76 |
| D | Paraffine 1 | Limonène | 3,5 | 24,1 | 72,3 | 6,3 | 2,99 |
| E | Paraffine 1 | Limonène | 4,3 | 23,9 | 71,8 | 6,2 | 1,85 |
| F | Paraffine 1 | Limonène | 5,3 | 23,7 | 71,0 | 6,1 | 1,56 |
| G | Paraffine 1 | Citronellal | 3,5 | 24,1 | 72,3 | 6,3 | 1,82 |
| H | Paraffine 2 | Limonène | 3,5 | 24,1 | 72,3 | 6,2 | 2,79 |
| I | Nacol ether 18 (Sasol) | Limonène | 3,5 | 24,1 | 72,3 | 6,3 | 3,20 |
| J | Paraffine 1 | Acetate de géranyle | 3,5 | 24,1 | 72,3 | 6,1 | 2,88 |
| K | Paraffine 1 | Limonène | 2,7 | 24,3 | 73,0 | 6,3 | 6,09 |
| L | Paraffine 1 | Limonène | 3,5 | 14,5 | 82,0 | 6,7 | 2,84 |
| M | Paraffine 1 | Limonène | 2,2 | 24,4 | 73,3 | 6,5 | 5,84 |
| N | Paraffine 1 | Limonène | 3,5 | 33,8 | 62,7 | 6,4 | 3,20 |

| | | | | | | | |
|---|---|---|---|---|---|---|---|
| Paraffine 1 : Paraffin wax 327204 de chez Sigma-Aldrich T_{f} = 53-57°C Paraffine 2 : Paraffin wax 411663 de chez Sigma-Aldrich T_{f} > 65°C | | | | | | | |

L'essai A représente l'art antérieur (absence d'un matériau à changement de phase). Les autres essais sont selon l'invention.

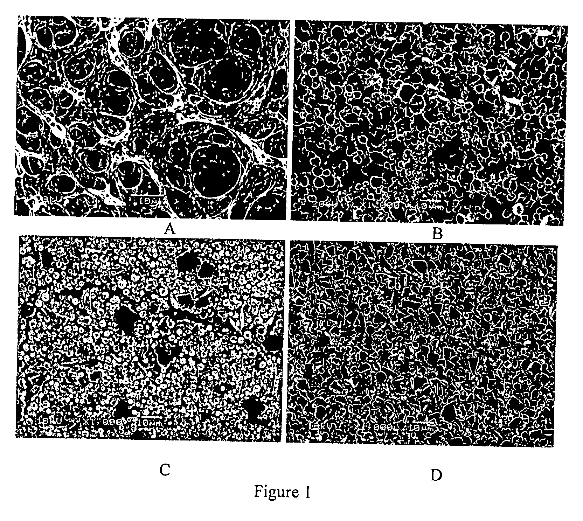

Il est observé que la présence d'un matériau à changement de phase (ou cire) améliore la résistance mécanique des capsules (fig.1). La figure 1A (en haut, à gauche : essai A) montre que les particules dépourvues de cire n'ont pas résisté au séchage et à la métallisation sous vide des échantillons (pas de particules ou résidus de particules visibles). Ces particules ne présentent pas la résistance mécanique minimale nécessaire au maintien de leur intégrité.

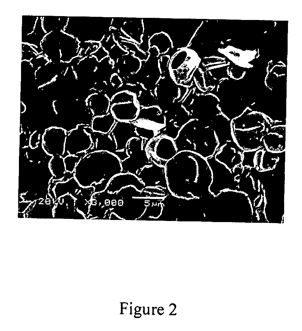

Par contre, les particules selon la présente invention (fig. 1A, 1B et 1C, respectivement essai B, essai 1 et essai H) ont maintenues leur intégrité après séchage et métallisation sous vide (qui sont des conditions très destructives). La figure 2 montre des microparticules de la présente invention obtenues selon l'essai B.

### Etude de la résistante des particules au cisaillement

La résistance des particules au cisaillement est évaluée grâce au D_{50%} (Mastersizer 2000 Malvern) des dispersions à teneur en particules à 47 ±1 % cisaillées dans une cellule double Couette du rhéomètre HAAKE Mars III (cisaillement durant 120s à 25°C). On représente la variation de D_{50%} / D⁰_{50%} en fonction du gradient de cisaillement appliqué (D⁰_{50%} correspond au D_{50%} de la dispersion de particules non cisaillée). Quand D_{50%} / D⁰_{50%} diminue, cela signifie que l'intégrité des particules n'est pas maintenu. On mesure alors au granulomètre laser des particules qui sont en fait des résidus de particules.

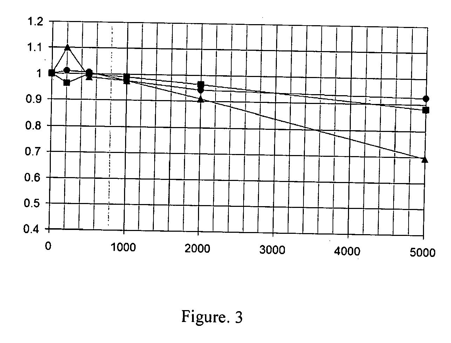

La figure 3 permet d'évaluer l'influence de la teneur en polymère HASE1 sur la résistance au cisaillement des microparticules (D_{50%} / D⁰_{50%} en fonction du gradient de cisaillement appliqué pour différents essais, ronds = essai C, carrés = essai M, triangles = essai K). On montre que plus la teneur en HASE est faible, moins les microparticules résistent au cisaillement.

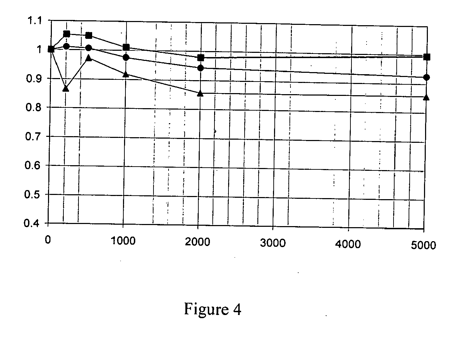

La figure 4 permet d'évaluer l'influence de la teneur en cire sur la résistance au cisaillement des microparticules (D_{50%} / D⁰_{50%} en fonction du gradient de cisaillement appliqué pour différents essais, carrés = essai L, ronds = essai C, triangles = essai N).

On montre que plus la teneur en cire est élevée, moins les microparticules résistent au cisaillement.

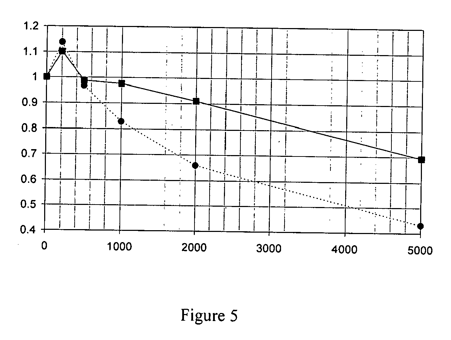

La figure 5 permet d'évaluer l'influence du pH final des dispersions sur la résistance au cisaillement des microparticules (D_{50%} / D⁰_{50%} en fonction du gradient de cisaillement appliqué pour l'essai K, carrés =la dispersion à un pH de 6.1, ronds : pH=la dispersion à un pH de 11,7).

## Revendications

1. Microparticules à enveloppe polymérique, comprenant :
a. au moins un copolymère acrylique de type HASE comprenant :
- au moins un monomère anionique présentant une fonction vinylique polymérisable et un groupement carboxyle ;
- au moins un monomère hydrophobe non ionique présentant une fonction vinylique polymérisable ; et
- au moins un macromonomère associatif oxyalkylé présentant une fonction vinylique polymérisable et une chaîne hydrocarbonée hydrophobe.
b. au moins un matériau à changement de phase solide-liquide présentant une température de transition de phase variant de 20 à 90°C ; et
c. au moins un agent actif.

2. Microparticules selon la revendication 1, selon lesquelles ledit matériau à changement de phase est choisi parmi les cires d'origine naturelle ou synthétique.

3. Microparticules selon l'une des revendications précédentes, selon lesquelles ledit copolymère acrylique de type HASE comprend, par rapport au poids total dudit copolymère :
- de 20% à 65% en poids d'au moins un monomère anionique présentant une fonction vinylique polymérisable et un groupement carboxyle ;
- de 35% à 75% en poids d'au moins un monomère hydrophobe non ionique présentant une fonction vinylique polymérisable ; et
- de 0,5% à 15% en poids d'au moins un macromonomère associatif oxyalkylé présentant une fonction vinylique polymérisable et présentant une chaîne hydrocarbonée hydrophobe.

4. Microparticules selon la revendication 1,2 ou 3, dans lesquelles ledit au moins un macromonomère associatif oxyalkylé dudit copolymère acrylique de type HASE présente une formule (I) :
(I) R-A-R'
dans laquelle :
- A représente une chaîne polymérique constituée de :
- m motifs d'oxyde d'alkylène de formule -CH₂CHR₁O- avec R₁ représentant un groupement alkyle comprenant de 1 à 4 carbones, et m variant de 0 à 150,
- p motifs d'oxyde d'alkylène de formule -CH₂CHR₂O- avec R₂ représentant un groupement alkyle comprenant de 1 à 4 carbones, et p variant de 0 à 150,
- n motifs d'oxyde d'éthylène avec n variant de 0 à 150,
dans laquelle m+n+p >4 et dans laquelle les motifs d'oxyde d'alkylène de formule -CH₂CHR₁O-, les motifs d'oxyde d'alkylène de formule-CH₂CHR₂O- et les motifs d'oxyde d'éthylène sont en bloc, alternés ou statistiques ;
- R représente un radical contenant une fonction insaturée polymérisable, appartenant au groupe des esters acrylique, méthacrylique, maléique, itaconique, ou crotonique ; et
- R' représente une chaîne hydrocarbonée linéaire, branchée ou ramifiée comprenant de 6 à 40 atomes de carbone ou un groupement cycloalkyle ou aryle substitué, ou non substitué, comprenant de 6 à 100 atomes de carbone.

5. Microparticules selon la revendication 4, dans lesquelles le copolymère acrylique de type HASE comprend au moins un macromonomère associatif oxyalkylé de formule (I) dans laquelle n varie de 15 à 150.

6. Microparticules selon l'une des revendications 4 ou 5, dans lesquelles ladite fonction R' dudit macromonomère associatif oxyalkylé de formule (I) représente une chaîne hydrocarbonée linéaire, branchée ou ramifiée comprenant de 8 à 20 atomes de carbone.

7. Microparticules selon l'une des revendications 4 à 6, dans lesquelles ledit macromonomère associatif oxyalkylé de formule (I) est tel que :
- m et p représentent respectivement 0,
- n varie de 15 à 150,
- R représente un radical contenant une fonction insaturée polymérisable appartenant au groupe des esters acryliques ou méthacryliques, et
- R' représente une chaîne hydrocarbonée linéaire comprenant de 8 à 18 atomes de carbone ou une chaîne alkyle branchée ou ramifiée comprenant de 12 à 20 atomes de carbone.

8. Microparticules selon l'une des revendications 1 à 7, comprenant, par rapport au poids total des microparticules :
a. de 1 à 20% en poids dudit au moins un copolymère acrylique de type HASE;
b. de 4 à 95% en poids d'au moins un matériau à changement de phase choisi parmi les cires d'origine naturelle ou synthétique ; et
c. de 4 à 95% en poids d'au moins un agent actif choisi parmi les parfums ou molécules odorantes.

9. Dispersion aqueuse comprenant des microparticules selon l'une des revendications précédentes.

10. Utilisation de microparticules selon les revendications 1 à 8 ou de dispersions aqueuses de microparticules selon la revendication 9 pour libérer un agent actif en réponse à un changement de pH, un changement de température et/ou à des frictions.

11. Utilisation de microparticules selon les revendications 1 à 8 ou de dispersions aqueuses de microparticules selon la revendication 9 pour la préparation de produits cosmétiques, agrochimiques, peintures, textiles, détergents, papier.

12. Procédé de préparation d'une dispersion aqueuse de microparticules à enveloppe polymérique, comprenant les étapes suivantes :
a) préparation d'une solution aqueuse comprenant :
- au moins un copolymère acrylique de type HASE solubilisé dans ladite solution aqueuse au moyen d'une base, ledit copolymère acrylique de type HASE comprenant :
- au moins un monomère anionique présentant une fonction vinylique polymérisable et un groupement carboxyle ;
- au moins un monomère hydrophobe non ionique présentant une fonction vinylique polymérisable ; et
- au moins un macromonomère associatif oxyalkylé présentant une fonction vinylique polymérisable et une chaîne hydrocarbonée hydrophobe ;
- au moins un agent actif ; et
- au moins un matériau à changement de phase solide-liquide présentant une température de transition de phase variant de 20 à 90°C, ledit matériau à changement de phase solide-liquide étant présent, ou ayant été introduit, dans la solution aqueuse à une température supérieure à sa température de transition de phase ; et
b) coacervation du polymère acrylique de type HASE pour conduire auxdites dispersions aqueuses de microparticules.

13. Procédé selon la revendication 12, dans lequel l'étape a) est réalisée selon les étapes suivantes :
a1) préparation d'une solution aqueuse comprenant le copolymère acrylique de type HASE solubilisé au moyen d'une base ;
a2) préparation d'un mélange comprenant l'agent actif et le matériau à changement de phase, ledit mélange étant préparé à une température supérieure à la température de transition de phase du matériau à changement de phase ; et
a3) introduction du mélange obtenu à l'étape a2) dans la solution aqueuse obtenue à l'étape a1).

14. Procédé selon la revendication 12, dans lequel l'étape a) est réalisée selon les étapes suivantes :
a1) préparation d'une solution aqueuse comprenant une base, au moins un agent actif, au moins un matériau à changement de phase et au moins un copolymère acrylique de type HASE ; et
a2) chauffage de la solution aqueuse obtenue à l'étape a1) à une température supérieure à la température de transition du matériau à changement de phase.

15. Procédé selon l'une quelconque des revendications 12 à 14, dans lequel la quantité de base utilisée pour solubiliser le copolymère est telle que le pH de la solution est supérieur ou égal à 6,5.

16. Procédé selon l'une quelconque des revendications 12 à 15, dans lequel l'étape b) est réalisée par ajout d'un acide, en quantité telle que le pH de la dispersion est inférieure ou égal à 6,5.

17. Procédé de préparation de microparticules à enveloppe polymérique solides, comprenant les étapes suivantes :
a) préparation d'une dispersion aqueuse de microparticules selon l'une des revendications 12 à 16 ;
b) séchage de la dispersion aqueuse.

## Patentansprüche

1. Mikropartikel mit Polymerhülle, umfassend:
a. mindestens ein Acrylcopolymer vom HASE-Typ, umfassend:
- mindestens ein anionisches Monomer mit einer polymerisierbaren Vinylfunktion und einer Carboxylgruppe;
- mindestens ein nichtionisches hydrophobes Monomer mit einer polymerisierbaren Vinylfunktion; und
- mindestens ein oxyalkyliertes assoziatives Makromonomer mit einer polymerisierbaren Vinylfunktion und einer hydrophoben Kohlenwasserstoffkette;
b. mindestens ein Fest-Flüssig-Phasenwechselmaterial mit einer Phasenübergangstemperatur von 20 bis 90°C; und
c. mindestens einen Wirkstoff.

2. Mikropartikel nach Anspruch 1, wobei das Phasenwechselmaterial aus Wachsen natürlicher oder synthetischer Herkunft ausgewählt ist.

3. Mikropartikel nach einem der vorhergehenden Ansprüche, wobei das Acrylcopolymer vom HASE-Typ, bezogen auf das Gesamtgewicht des Copolymers:
- 20 bis 65 Gew.-% mindestens eines anionischen Monomers mit einer polymerisierbaren Vinylfunktion und einer Carboxylgruppe;
- 35 bis 75 Gew.-% mindestens eines nichtionischen hydrophoben Monomers mit einer polymerisierbaren Vinylfunktion; und
- 0,5 bis 15 Gew.-% mindestens eines oxyalkylierten assoziativen Makromonomers mit einer polymerisierbaren Vinylfunktion und einer hydrophoben Kohlenwasserstoffkette umfasst.

4. Mikropartikel nach Anspruch 1, 2 oder 3, wobei das mindestens eine oxyalkylierte assoziative Makromonomer des Acrylcopolymers vom HASE-Typ die Formel (I) aufweist:
(I) R-A-R'
in der:
- A für eine Polymerkette aus:
- m Alkylenoxid-Einheiten der Formel -CH₂CHR₁O-, wobei R₁ für eine Alkylgruppe mit 1 bis 4 Kohlenstoffatomen steht und m 0 bis 150 beträgt,
- p Alkylenoxid-Einheiten der Formel -CH₂CHR₂O-, wobei R₂ für eine Alkylgruppe mit 1 bis 4 Kohlenstoffatomen steht und p 0 bis 150 beträgt,
- n Ethylenoxid-Einheiten, wobei n 0 bis 150 beträgt, steht, wobei m+n+p >4 und wobei die Alkylenoxid-Einheiten der Formel -CH₂CHR₁O-, die Alkylenoxid-Einheiten der Formel -CH₂CHR₂O- und die Ethylenoxid-Einheiten blockförmig, alternierend oder statistisch angeordnet sind;
- R für einen Rest mit einer polymerisierbaren ungesättigten Funktion aus der Gruppe der Acrylsäure-, Methacrylsäure-, Maleinsäure-, Itaconsäure- oder Crotonsäureester steht; und
- R' für eine lineare, verzweigte oder verästelte Kohlenwasserstoffkette mit 6 bis 40 Kohlenstoffatomen oder eine substituierte oder unsubstituierte Cycloalkyl- oder Arylgruppe mit 6 bis 100 Kohlenstoffatomen steht.

5. Mikropartikel nach Anspruch 4, wobei das Acrylcopolymer vom HASE-Typ mindestens ein oxyalkyliertes assoziatives Makromonomer der Formel (I), in der n 15 bis 150 beträgt, umfasst.

6. Mikropartikel nach einem der Ansprüche 4 oder 5, wobei die Funktion R' des oxyalkylierten assoziativen Makromonomers der Formel (I) für eine lineare, verzweigte oder verästelte Kohlenwasserstoffkette mit 8 bis 20 Kohlenstoffatomen steht.

7. Mikropartikel nach einem der Ansprüche 4 bis 6, wobei das oxyalkylierte assoziative Makromonomer der Formel (I) so beschaffen ist, dass:
- m und p jeweils für 0 stehen,
- n 15 bis 150 beträgt,
- R für einen Rest mit einer polymerisierbaren ungesättigten Funktion aus der Gruppe der Acrylsäure- oder Methacrylsäureester steht und
- R' für eine lineare Kohlenwasserstoffkette mit 8 bis 18 Kohlenstoffatomen oder eine verzweigte oder verästelte Alkylkette mit 12 bis 20 Kohlenstoffatomen steht.

8. Mikropartikel nach einem der Ansprüche 1 bis 7, umfassend, bezogen auf das Gesamtgewicht der Mikropartikel:
a. 1 bis 20 Gew.-% mindestens eines Acrylcopolymers vom HASE-Typ;
b. 4 bis 95 Gew.-% mindestens eines Phasenwechselmaterials, das aus Wachsen natürlicher oder synthetischer Herkunft ausgewählt ist; und
c. 4 bis 95 Gew.-% mindestens eines Wirkstoffs, der aus Parfümen oder Geruchsmolekülen ausgewählt ist.

9. Wässrige Dispersion, umfassend Mikropartikel nach einem der vorhergehenden Ansprüche.

10. Verwendung von Mikropartikeln nach den Ansprüchen 1 bis 8 oder wässrigen Dispersionen von Mikropartikeln nach Anspruch 9 zur Freisetzung eines Wirkstoffs als Reaktion auf eine pH-Wert-Änderung, eine Temperaturänderung und/oder Reibung.

11. Verwendung von Mikropartikeln nach den Ansprüchen 1 bis 8 oder wässrigen Dispersionen von Mikropartikeln nach Anspruch 9 zur Herstellung von kosmetischen Produkten, agrochemischen Produkten, Anstrichmittelprodukten, Textilprodukten, Wasch- oder Reinigungsmittelprodukten oder Papierprodukten.

12. Verfahren zur Herstellung einer wässrigen Dispersion von Mikropartikeln mit Polymerhülle, das folgende Schritte umfasst:
a) Herstellung einer wässrigen Lösung, umfassend:
- mindestens ein Acrylcopolymer vom HASE-Typ, das in der wässrigen Lösung mit Hilfe einer Base solubilisiert ist, wobei das Acrylcopolymer vom HASE-Typ Folgendes umfasst:
- mindestens ein anionisches Monomer mit einer polymerisierbaren Vinylfunktion und einer Carboxylgruppe;
- mindestens ein nichtionisches hydrophobes Monomer mit einer polymerisierbaren Vinylfunktion; und
- mindestens ein oxyalkyliertes assoziatives Makromonomer mit einer polymerisierbaren Vinylfunktion und einer hydrophoben Kohlenwasserstoffkette;
- mindestens einen Wirkstoff; und
- mindestens ein Fest-Flüssig-Phasenwechselmaterial mit einer Phasenübergangstemperatur von 20 bis 90°C, wobei das Fest-Flüssig-Phasenwechselmaterial bei einer über seiner Phasenübergangstemperatur liegenden Temperatur in der wässrigen Lösung vorliegt oder in die wässrige Lösung eingetragen worden ist; und
b) Koazervation des Acrylpolymers vom HASE-Typ, was die wässrigen Dispersionen von Mikropartikeln ergibt.

13. Verfahren nach Anspruch 12, bei dem der Schritt a) gemäß den folgenden Schritten durchgeführt wird:
a1) Herstellung einer wässrigen Lösung, die das mit Hilfe einer Base solubilisierte Acrylcopolymer vom HASE-Typ umfasst;
a2) Herstellung einer Mischung, die den Wirkstoff und das Phasenwechselmaterial umfasst, wobei die Mischung bei einer über der Phasenübergangstemperatur des Phasenwechselmaterials liegenden Temperatur hergestellt wird; und
a3) Eintragen der in Schritt a2) erhaltenen Mischung in die in Schritt a1) erhaltene wässrige Lösung.

14. Verfahren nach Anspruch 12, bei dem der Schritt a) gemäß den folgenden Schritten durchgeführt wird:
a1) Herstellung einer wässrigen Lösung, die eine Base, mindestens einen Wirkstoff, mindestens ein Phasenwechselmaterial und mindestens ein Acrylcopolymer vom HASE-Typ umfasst; und
a2) Erhitzen der in Schritt a1) erhaltenen wässrigen Lösung auf eine über der Übergangstemperatur des Phasenwechselmaterials liegende Temperatur.

15. Verfahren nach einem der Ansprüche 12 bis 14, bei dem die für die Solubilisierung des Copolymers verwendete Basenmenge derart bemessen wird, dass der pH-Wert der Lösung größer gleich 6,5 ist.

16. Verfahren nach einem der Ansprüche 12 bis 15, bei dem der Schritt b) durch Zugabe einer Säure in einer solchen Menge, dass der pH-Wert der Dispersion kleiner gleich 6,5 ist, durchgeführt wird.

17. Verfahren zur Herstellung von festen Mikropartikeln mit Polymerhülle, das folgende Schritte umfasst:
a) Herstellung einer wässrigen Dispersion von Mikropartikeln nach einem der Ansprüche 12 bis 16;
b) Trocknung der wässrigen Dispersion.

## Claims

1. Polymeric-enveloped microparticles, containing:
a. at least one acrylic copolymer of the HASE type containing:
- at least one anionic monomer having a polymerizable vinyl function and a carboxyl group,
- at least one non-ionic hydrophobic monomer having a polymerizable vinyl function and
- at least one oxyalkylated associative macro monomer having a polymerizable vinyl function and a hydrophobic hydrocarbon chain,
b. at least one solid-liquid phase change material having a phase transition temperature varying from 20 to 90°C and
c. at least one active agent.

2. Microparticles according to claim 1, according to which said phase change material is chosen from the natural or synthetic waxes.

3. Microparticles according to one of the preceding claims, according to which said HASE type acrylic copolymer contains, relative to the total weight of said copolymer:
- from 20% to 65% by weight of at least one anionic monomer having a polymerizable vinyl function and a carboxyl group,
- from 35% to 75% by weight of at least one non-ionic hydrophobic monomer having a polymerizable vinyl function and
- from 0.5% to 15% by weight of at least one oxyalkylated associative monomer having a polymerizable vinyl function and having a hydrophobic hydrocarbon chain.

4. Microparticles according to claim 1, 2 or 3, in which said at least one oxyalkylated associative macromonomer of said HASE type acrylic copolymer has a formula (I):
(I) R-A-R'
in which:
- A represents a polymeric chain consisted of:
- m units of alkylene oxide of formula -CH₂CHR₁O- with R₁ representing an alkyl group containing from 1 to 4 carbons and m varying from 0 to 150,
- p units of alkylene oxide of formula -CH₂CHR₂O- with R₂ representing an alkyl group containing from 1 to 4 carbons and p varying from 0 to 150 and
- n units of ethylene oxide with n varying from 0 to 150, in which m+n+p > 4 and in which the units of alkylene oxide of formula
- CH₂CHR₁O-, the units of alkylene oxide of formula -CH₂CHR₂O- and the units of ethylene oxide are in a block, alternated or are random,
- R represents a radical containing a polymerizable unsaturated function belonging to the group of acrylic, methacrylic, maleic, itaconic or crotonic esters and
- R' represents a linear, connected or branched hydrocarbon chain containing from 6 to 40 carbon atoms or a substituted or non-substituted cycloalkyl or aryl group containing from 6 to 100 carbon atoms.

5. Microparticles according to claim 4, in which the HASE type acrylic copolymer contain at least one oxyalkylated associative macromonomer of formula (I) in which n varies from 15 to 150.

6. Microparticles according to one of claims 4 or 5, in which said function R' of said oxyalkylated associative macromonomer of formula (I) represents a linear, connected or branched hydrocarbon chain containing from 8 to 20 carbon atoms.

7. Microparticles according to one of claims 4 to 6, in which said oxyalkylated associative macromonomer of formula (I) is such that:
- m and p represent respectively 0,
- n varies from 15 to 150,
- R represents a radical containing a polymerizable unsaturated function belonging to the group of acrylic or methacrylic esters and
- R' represents a linear hydrocarbon chain containing from 8 to 18 carbon atoms, or a connected or branched alkyl chain containing from 12 to 20 carbon atoms.

8. Microparticles according to one of claims 1 to 7, containing, relative to the total weight of the microparticles:
a. from 1 to 20% by weight of said at least one HASE type acrylic copolymer,
b. from 4 to 95% by weight of at least one phase change material chosen from natural or synthetic waxes and
c. from 4 to 95% by weight of at least one active agent chosen from perfumes or odorous molecules.

9. Aqueous dispersion containing microparticles according to any one of the preceding claims.

10. Use of microparticles according to claims 1 to 8 or of aqueous dispersions of microparticles according to claim 9 to release an active agent in response to a change in pH, a change in temperature and/or to frictions.

11. Use of microparticles according to claims 1 to 8 or of aqueous dispersions of microparticles according to claim 9 for the preparation of cosmetic, agrochemical, paint, textile, detergent or paper products.

12. Process for the preparation of an aqueous dispersion of polymeric-enveloped microparticles containing the following steps:
a) preparation of an aqueous solution containing:
- at least one HASE type acrylic copolymer solubilised in said aqueous solution by means of a base, said HASE type acrylic copolymer containing:
- at least one anionic monomer having a polymerizable vinyl function and a carboxyl group,
- at least one non ionic hydrophobic monomer having a polymerizable vinyl function and
- at least one oxalkylated associative monomer having a polymerizable vinyl function and a hydrophobic hydrocarbon chain,
- at least one active agent and
- at least one solid-liquid phase change material having a phase transition temperature varying from 20 to 90°C, said solid-liquid phase change material being present, or having been introduced, into the aqueous solution at a temperature that is greater than its phase transition temperature and
b) coacervation of the HASE type acrylic polymer to lead to said aqueous dispersions of microparticles.

13. Process according to claim 12, in which step a) is carried out according to the following steps:
a1) preparation of an aqueous solution containing the HASE type acrylic copolymer solubilised by means of a base,
a2) preparation of a mixture containing the active agent and the phase change material, the said mixture being prepared at a temperature greater than the phase transition temperature of the phase change material and
a3) introduction of the mixture obtained in step a2) into the aqueous solution obtained in step a1).

14. Process according to claim 12, in which step a) is carried out according to the following steps:
a1) preparation of an aqueous solution containing a base, at least one active agent, at least one phase change material and at least one HASE type acrylic copolymer and
a2) heating of the aqueous solution obtained in step a1) to a temperature greater than the transition temperature of the phase change material.

15. Process according to any one of the claims 12 to 14, in which said amount of base used to solubilise the copolymer is such that the pH of the solution is greater than or equal to 6.5.

16. Process according to any one of the claims 12 to 15, in which step b) is carried out by the addition of an acid in an amount such that the pH of the dispersion is less than or equal to 6.5.

17. Process for the preparation of solid polymeric-enveloped microparticles containing the following steps:
a) preparation of an aqueous dispersion of microparticles according to any one of the claims 12 to 16 and
b) drying of the aqueous dispersion.
